(19) **Europäisches Patentamt**

**European Patent Office**

**Office européen des brevets**

(11) **EP 1 326 183 A1**

(12) **EUROPEAN PATENT APPLICATION**

(43) Date of publication:
**09.07.2003 Bulletin 2003/28**

(51) Int Cl.⁷: **G06F 17/30**, G06F 19/00

(21) Application number: **02290037.7**

(22) Date of filing: **08.01.2002**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI** | (72) Inventor: **The designation of the inventor has not yet been filed** |
| (71) Applicant: **SYNT:EM S.A.**<br>**30000 Nîmes (FR)** | (74) Representative: **Brasnett, Adrian Hugh et al**<br>**Mewburn Ellis**<br>**York House**<br>**23 Kingsway**<br>**London WC2B 6HP (GB)** |

(54) **Method for silico screening of molecules**

(57) The invention provides a method for establishing a high-throughput virtual screening system, which method comprises providing an initial database of molecular structures which comprise active compounds which exhibit a desired activity and inactive compounds which do not exhibit a desired activity calculating descriptor values for a plurality of descriptors for said compounds and selecting a set of descriptors, ADS, associated with compounds exhibiting said activity and a second set of descriptors, IDS, associated with compounds not exhibiting said activity, said second set of descriptors being different from said first set.

**Description**

Field of the Invention.

The present invention relates to a method of *in silico* screening for compounds having a desired activity using a double filtering technique.

Background to the Invention.

A key process in the pharmaceutical industry is the identification of new lead compounds. Such compounds should ideally be somewhat different to existing drugs in terms of backbone structure and must be sensible starting points for further chemistry and design effort in the drug development process. A lead compound will have certain minimal properties that identify it as a potentially useful compound but requires further optimisation of those properties before it can be considered a worthwhile candidate for a drug development programme. In the last ten years or so there has been much and continuing development within the pharmaceutical industry of technologies enabling the rapid, automated screening of very large numbers of compounds against a biological target of some kind. This approach to new lead discovery is usually referred to as high-throughput screening (HTS). Concomitant to HTS is the availability of compound collections or the means to synthesize the large numbers of compounds to be tested. In general, compounds are synthesized using combinatorial chemistry (CombiChem) techniques wherein the substituents around a common backbone are varied in a systematic manner and/or according to the reagents available taking into account synthesis and cost constraints. More recently, developments in multi-component reaction (MCR) chemistry [Dömling and Ugi, 2000] have promised greater diversity in the backbone structures obtained although, like CombiChem in general, the potential of this approach has yet to be fully realised.

At an early stage in the development of HTS/CombiChem it rapidly became clear that, whilst it may be possible to screen (synthesize and test) perhaps a few million compounds within the context of a given research programme, such a number is a small fraction of the total possible number of drug candidates. It is difficult to estimate exactly but a conservative estimate is that there may be $10^{13}$ possible drug-like candidates [Walters *et al.*, 1998; Labute, 1999]. It is obvious that at a typical rate of $10^5$ per day that $10^8$ days or roughly 270,000 years would be required to test all these compounds and this is when only one biological target is considered; even scaling this up by a factor of 1,000 leaves an impossible task. The other main factor involved is the cost which clearly becomes prohibitive even if this is only $5 per measurement [Labute, 1999]. Whilst there is continuing improvement of the throughput and cost of HTS (better robotics, miniaturisation of samples to nanolitre levels etc.) [Dove, 1999] there is a clear need to reduce to manageable levels the numbers of compounds actually synthesized and tested. At the same time there has also been a realisation that "more" does not necessarily mean "better"; that is to say, it is not merely the numbers of screened compounds that need to be reduced but that these compounds must be the right ones. Thus, it is with both these considerations in mind that pharmaceutical companies have turned to computational techniques to improve the hit-rate from HTS programs; these methods are variously referred to as library design, *in silico screening* or *virtual screening.*

The *in silico* screening or *virtual* screening (ISS or VS) techniques [Walters *et al,* 1998.] coupled with *virtual* compound synthesis (or enumeration) provide a means of selecting compounds to be synthesized and tested in an rational manner. The ISS methods fall into two non-mutually exclusive categories.

The first approach is based solely upon diversity analysis and can be used where there is no knowledge, direct or indirect, of the structural features required for binding to the target concerned. The premise of the diversity-based methods is that the selection of a diverse subset of molecules which in some way cover or represent the structure and/or properties of a much larger, but defined, set of structures provides a better than random chance of finding molecules active for a given target. Typically, the compounds selected may also be required to satisfy general, drug-like constraints [Oprea, 2000; Walters *et al*, 1999.; Frimurer *et al.,* 2000] and/or exhibit features known to favour, for example, oral bioavailability, such as those defined by the Lipinski rules [Lipinski *et al.,* 1997].

The second, more targeted, approach to ISS relies upon the availability of some information about a ligand binding site. This may be *direct* information as given, for example, by x-ray crystallographic analysis of ligand-receptor complexes or by NMR analysis and, based on these data, *de novo* design of new ligands may be performed. Alternatively, such direct information may not be available and one may use the *indirect* information that can be extracted from a series of known active ligands; typically this involves the generation of a 3D pharmacophore hypothesis [Guner, 2000] that can be tested using statistical methods such as Comparative Molecular Field Analysis [Cramer *et al.,* 1988]. In either case the information provided may be used to bias the design of virtual combinatorial libraries and thence to virtually screen such libraries. Virtual screening may be done using high-throughput docking-based approaches - for example, around 1 million compounds have recently been screened [Protherics] albeit using a large number of high performance processors in parallel. Alternatively, pharmacophoric queries may be developed from either direct or indirect sources [Guner, 2000] and 3D searches undertaken for structures able to match the proposed pharmacophore.

The problem when dealing with 3D structural descriptions is that a molecule can usually adopt many different conformations and may also exist in different enantiomeric forms. This means that for each molecule many different 3D arrangements of its atoms have to be considered when performing a pharmacophore search or high throughput docking screen. So, whilst the 3D-information-based methods can be very effective at identifying potential hit structures they are computationally demanding and cannot be routinely applied to the virtual screening of millions of compounds without the use of huge computational resources. Clearly, even the one million compounds screened by Protherics is only a minute fraction of a drug-universe of $10^{13}$ compounds. There is therefore much interest in methods which, in conjunction with library design techniques, enable the efficient and effective ISS of very large chemical structure databases. In this context efficiency refers to the speed at which the method can be applied while effectiveness refers to the quality of the molecules selected In order to be efficient ISS methods rely upon rapidly-computable, conformation-independent 2D descriptions of molecular structure which include topological descriptors and various binary bitstring descriptors. In a number of validation studies of effectiveness these types of descriptor have been shown to be surprisingly effective at distinguishing drug-like compounds from non-drugs and at separating structures with different biological activity [Brown and Martin, 1996, 1998; Matter, 1997]. These studies are based upon whole molecule similarity comparisons using 2D descriptors wherein unsupervised cluster analysis techniques were used to group similar compounds together. These studies maybe criticised in the sense that there were probably many compounds with the same scaffold present in the databases analysed [Briem and Lessel, 2000]; this is typical of in-house compound collections, where congeneric series of molecules will have been synthesized and tested, and is inherent to combinatorially generated libraries of compounds. As such this is clearly an ideal situation for topological descriptors to perform well, since a given scaffold will always generate the same set of 2D descriptors and these descriptors will often make a significant contribution to any whole molecule similarity calculation. The extent of this contribution will of course depend upon the size of the scaffold relative to the whole molecule but it is clear that, where the objective is to scaffold-hop (*i. e.*, to cluster or identify compounds with different scaffolds but similar biological activity) whole molecule comparisons are often not the ideal approach. One solution to this problem is to select a small subset of descriptors which are both scaffold insensitive and in some way capture the molecular features which confer drug-like characteristics and/or put the molecule into a particular activity class. This approach requires both a technique for descriptor selection and a training set of compounds with various scaffolds to work with.

There are a number of published virtual screening methods which make use of 2D descriptors coupled with methods to model differences between active and inactive compounds and thence make predictions for new or unseen compounds. However very few of them appear to have been used with very large numbers of compounds and even fewer have been fully validated in terms of assay results with novel structures selected by a virtual screening process.

Gálvez and co-workers have described the use of classical stepwise linear discriminant analysis (LDA) to separate active/inactive compounds using topological descriptors [Gálvez *et al.,* 1994] and have described the effective screening of available compound databases [Gálvez *et al.,* 1995] as well as virtual screening databases designed to be biased toward a known target [de Julián-Ortiz *et al.,* 1999]. In the last case four separate LDA were developed which individually model either binding data (anti-HSV-1 activity expressed as $IC_{50}$ values) or one of three forms of pharmacokinetic data - in the final analysis predicted actives must satisfy all such functions. A library of virtual structures was generated, consisting of phenol esters and anilides, and this was screened using the four discriminant models. Five new closely related structures were selected, synthesized and tested; the structures had activities in the range 0.9 to 1.8 $\mu$M. In another example non-linear discriminant analysis has been performed using neural networks [García-Domenech and de Julián-Ortiz, 1998] and in this instance the non-linear method was found to outperform classical LDA albeit solely on the basis of training/test set statistics; *i.e.*, virtual screening was not carried out. As ever with neural network (NN) models there is the drawback that it is difficult for the user to interpret the model obtained given the complex internal weighted connectivity structure of NNs. Gálvez *et al*. [1996] also describe the use of so-called pharmacological distribution diagrams (PDDs), essentially plots of the number of active and inactive compounds falling within particular intervals of a predicted dependent variable.

The TOSS-MODE approach [Estrada *et al.,* 2000] is also based upon topological descriptors and, like Gálvez *et al.,* uses LDA to define a function to separate active from inactive compounds. The differences to the work of Gálvez *et al*. lie in the type of topological descriptors used and in the fact that *a posteriori* probabilities, rather than hard cutoffs, are used to classify compounds to be predicted as active/inactive. In validation studies the method has been applied successfully to the simulated virtual screening of anticancer compounds (Ras farnesyltransferase inhibitors). The screening database consisted of 30 structures previously screened, and shown to be active, against Ras FTase and were compounds structurally very different to those of the training set; 87% of these were correctly predicted active. This same model was also used to virtually screen a small selection of 20 already synthesized, or designed but not yet synthesized, carbonucleosides. After assay an 80% correct classification was obtained with 6 inactive compounds and 10 active compounds correctly classified by the model. Further studies with larger numbers of compounds and, in particular, a sufficiently large control set of inactives is required to properly validate the model since the possibility for false positives has not been fully addressed.

The QuaSAR-Binary method [Labute *et al*., 1999] makes use of 2D descriptors and applies Bayesian probability density functions for binary classification (active-inactive). The method is inherently non-linear. There are a number of published validation studies using datasets consisting of ligands with known activity [Hua Gao *et al.,* 1999] but there are no published true virtual screening results at present.

As an alternative to algorithms whose objective is classification QSAR equations, derived for example using multiple linear regression (MLR) or partial least squares to latent structures (PLS) regression, can be used for virtual screening. An approach has been described [Hoffman *et al.,* 2000] which uses MolConnZ topological descriptors [MolConnZ] and PLS coupled to GA (genetic algorithm)-driven variable selection to build QSAR models for dopamine transporter (DAT) inhibition, dopamine D1 antagonist affinity and serotonin ligand affinity. The DAT model was used to screen the National Cancer Institute (NCI) database [NCI]. A final selection (prioritisation) from the predicted actives was made by calculating a whole molecule similarity score to known actives and choosing those most similar to the latter; five molecules were selected as suitable for further development as novel DAT inhibitors with $IC_{50}$ values around $1\mu M$.

(WO 99/35598, Grassy *et al.,* 1999) describes a method, referred to as OASIS, which is intended for use as a high throughput virtual screening tool. OASIS relies upon cluster significance analysis (CSA) coupled to a genetic algorithm (GA) for descriptor selection. CSA operates by calculating the sum of squared distances (MSD) between all pairs of structures in a given class (the actives, for example) and compares this to the pairwise MSDs for all other possible subsets from the training set of compounds (*i.e.*, both actives and inactives). The proportion, *p*, of such subsets which have an MSD higher than the MSD for the active subset gives a statistical measure of the significance of the cluster of active molecules using a given set of descriptors. The GA is used to drive the selection of a subset of an initial, large pool of descriptors that provides the most statistically significant separation of actives from inactives (*i.e.*, a minimal p value is the objective function for the GA).

Disclosure of the Invention.

The present invention provides a novel approach to screening molecules, which is both simpler to operate than some of the above described prior art approaches and which provides a potentially higher level of hits. We have termed this approach "Diversifier-Discriminant Analysis", or "DIV-DA".

DIV-DA enables rapid and automated virtual screening of very large numbers of compounds for a desired activity against a given biological target, and starting from an initial database of diverse molecular structures (several scaffolds) experimentally found to be active or inactive (weakly active). In the DIV-DA approach, descriptors are calculated independently for active and inactive molecular structures, and screening is based on determining if a candidate compound both satisfies some and preferably all of the descriptors for the active structures, and also does not satisfy at least one of the descriptors for the inactive structures.

Accordingly; the present invention provides a method for establishing a high-throughput virtual screening system, which method comprises:

providing an initial database of molecular structures which comprise active compounds which exhibit a desired activity and inactive compounds which do not exhibit a desired activity;
calculating descriptor values for a plurality of descriptors for said compounds; and
selecting a set of descriptors, ADS associated with compounds exhibiting said activity and a second set of descriptors, IDS with compounds not exhibiting said activity, said second set of descriptors being different from said first set.

In the present invention, the term 'descriptor' refers to the final result of a logical and mathematical procedure which transforms chemical information encoded within a symbolic representation of a molecule into an useful number or the result of some standardized experiment (Todeschini & Consonni, 2000). Included within the definition of 'descriptor' can be calculated, or actual experimentally measured, physicochemical properties such as octanol-water partition coefficients, ionisation potentials, dipole moments and the like. The term is also considered to extend to experimental measurements of biological activity wherein, for example, biological affinity measurements for a panel of enzymes or receptors may be used to model and predict biological affinity for another enzyme or receptor. In the simplest case, a descriptor might be a count of the number of hydroxyl groups, but can also be and frequently are more complex functions associated with the topology or topography of a compound; this includes semi-empirical or quantum-mechanically derived descriptors. The descriptor value or set of values is the actual numerical value or set of values of a descriptor which can be derived for a particular compound.

The descriptor sets ADS and IDS are defined here as sets of descriptors which have defined value ranges for active and inactive compounds respectively. Thus, for example, if for a particular activity it was found that active compounds all had more than 2 hydroxyls groups but less than 10, and all inactive compounds have a value for this descriptor less than 2 and/or more than 10, then a candidate ADS member might include this descriptor (count of hydroxyls) having a range of values from 3 to 9.

In the invention, it will be understood that it is the ADS set of descriptors as a whole, that is different from the IDS set of descriptors, not simply in their values (as in prior art LDA methods), but also in the particular descriptors selected. However, it is possible for the two sets to have one or more descriptors in common, though there will not be a complete overlap, unlike in classical DA.

In a preferred aspect, the DIV-DA process can involve the following steps: *(i)* after calculation of all available descriptors, the definition of a training set and a test set from the initial database of compounds, based on descriptor distribution (discriminant profiles); both sets contain active and inactive compounds; *(ii)* model building using descriptor distribution information only for the training set, by a stepwise procedure, leading to two sets of descriptors, and to the definition of an application domain of the model; and *(iii)* using a double filtering technique, validation of the model with the test set. Then, with the same filtering technique, prediction for the desired activity can be made on large virtual libraries. As shown in the results section herein, the method is suitable for the identification of novel, potential lead structures (new scaffolds) and for enrichment in hit rates.

By *"application domain"*, it is meant the limits of the descriptors for each and every ADS and IDS descriptors. Generally, the model for virtual screening purposes is used for molecules having both all ADS and IDS descriptors values within the range defined by the training set.

Accordingly, a preferred aspect of the invention provides a method in which the selection of descriptors may be performed by:

(a) providing a plurality of candidate active and inactive compound descriptors and defining a range of values of each descriptor for both active and inactive subsets of compounds;
(b) dividing the initial database of compounds into a test set and a training set by a process which comprises;

(1.i) evaluating each active compound by determining its descriptor value for each candidate inactive descriptor;
(1.ii) selecting as a test set member each active compound whose descriptor value for each candidate inactive descriptor has a value lying within the range defined by inactive compounds and selecting as a training set member the remaining active compounds; and
(2.i) evaluating each inactive compound by determining its descriptor value for each candidate active descriptor;
(2.ii) selecting as a test set member each inactive compound whose descriptor value for each candidate active descriptor has a value lying within the range defined by active compounds and selecting as a training set member the remaining inactive compounds;

(c) calculating the range of values for each descriptor of active and inactive compounds in the training set; and
(d) selecting a plurality of descriptors and values thereof for the ADS and IDS based on the ability of the descriptors to discriminate active and inactive members of the training set.

It will be understood that in step (b) the evaluation of the active and inactive compounds may be performed sequentially in either order, or in parallel.

The ADS descriptor set may be selected on a stepwise basis in which each potential ADS descriptor is ranked for its degree of overlap with inactive compounds, a first descriptor with the least degree of overlap is selected, and the process is repeated to determine the next and subsequent descriptors until there is no further gain in their discriminatory effectiveness which allows removal of additional inactive compounds.

The same process may be used in selecting the IDS descriptor set.

The invention further provides a method of screening a compound for a putative desired activity, which method comprises providing a screening system established as described above and herein, calculating for the compound its descriptors for the ADS and IDS sets, and selecting a compound as putatively having said desired activity if all the ADS descriptors are satisfied and, at least, one IDS descriptor is not satisfied.

Compounds selected according to the invention may be synthesised and tested for the desired activity. In principle, any desired activity such as human pharmaceutical activity, veterinary activity, activity in the field of agriculture, such as pesticidal or herbicidal activity, and the like. The invention has particular application in the field of human pharmaceutical activity, and thus desired activities of compounds which may be screened can be selected from anti-viral activity, anti-bacterial activity, enzyme inhibitory activity, receptor-binding activity, activity related to inhibition of protein-protein interactions, activity mediated via allosteric mechanisms, non-toxicity to a human, anticancer activity, and anti-pain activity Those compounds exhibiting such activity can be prepared in the form of pharmaceutical compositions suitable for a method of treatment of a condition associated with the activity. Alternatively, the compounds may form the basis from which to develop further compounds with improved activity over the selected compound. Compounds selected by the methods of the invention, and formulations of such compounds, form a further aspect of the present invention.

These and other aspects of the invention are described in further detail below.

Description of the Drawings.

Figure 1A shows descriptor profiles preferred in classical linear discriminant analysis.

Figures 1B and 1C show descriptor profiles utilised by DIV-DA in *addition* to the classical linear discriminant profile (Fig. 1A); the profiles shown would make the descriptors appropriate for inclusion in the active descriptor set.

Figure 2 shows suitable descriptor profiles: the upper (dark grey) line in each plot is a representation of the descriptor range for active compounds while the lower (light grey) line is that for inactive compounds. Each profile is labelled according to whether it is suitable for inclusion in the ADS (ACT), IDS (INACT) or both descriptor sets (ACT&INACT) - the dotted lines define the active-inactive overlap region for a descriptor.

Figure 3 shows a summary of the process used to segregate the available active and inactive compounds into a training and a test set; a model is subsequently built using the training set and this is then validated by making active-inactive predictions for the test set.

Figure 4 shows a summary of the procedure used to select the first descriptor to be included in the ADS; an analogous approach is used for selecting the first IDS descriptor.

Figure 5 shows a summary of the procedure used to select the second and subsequent descriptors to be included in the ADS; an analogous approach is used for selecting IDS descriptors.

Detailed Description of the Invention.

A major difference between DIV-DA and the approaches of the prior art described above lies primarily in the nature of the descriptor selection technique which, whilst it permits descriptors with profiles suitable for classical DA, also allows the use of descriptors where, at the limit, there is no difference in the means for active-inactive descriptors; rather the active-inactive descriptors differ in their value ranges meaning that embedded distributions are permitted. This means that a DIV-DA model can encode factors not available to classical DA; *i.e.*, additional descriptors are available to DIV-DA. Indeed, analyses have shown that models based upon only descriptors with classical distributions perform less well than those permitted by DIV-DA.

In addition to this a preferred aspect of the invention is that DIV-DA may utilize a unique double filtering method wherein a compound predicted to be active must not only satisfy the *active* model but must not be predicted *inactive* by the inactive model. This process is in fact only meaningful where non-classical distributions of descriptors are permitted since for classical LDA only one set of descriptors would be used to separate actives from inactives while for DIV-DA the methodology allows for different sets of non-classically distributed descriptors to be selected for the active and the inactive descriptor sets. Double-filtering thus provides a second level of confidence in ones predictions. It results in the identification of two sets of 2D descriptors which are best able to separate the compounds by class:

1) ADS: Active class Descriptor Set: this is the set of descriptors which clusters active compounds and excludes inactive compounds; 2) IDS: Inactive class Descriptor Set: this is the set of descriptors which clusters inactive and excludes active compounds. The selection of descriptors is a stepwise procedure and the final model consists of two groups of descriptors (the ADS and the IDS) with defined ranges for the selected descriptors. When applying the model, a compound finally accepted as a predicted-active must both be predicted active by the ADS descriptors and also not predicted inactive when the IDS descriptors are examined; this double filtering approach is usually found to eliminate further compounds from consideration in comparison to a selection based solely on the ADS.

A further distinctive feature of this preferred embodiment of the DIV-DA process is that the means to identify compounds suitable for a training set and a test (validation) set is built into the procedure.

Thus the DIV-DA method described here is an ISS method based upon discriminant computational models derived solely from sets of ligands with activity data for a particular biological target (receptor or otherwise); direct receptor knowledge is not necessary. A training set is required from which to develop the class discriminant (or classification) function and this typically consists of active and inactive (or weakly active) compounds. In fact, DIV-DA also includes within its process the means to define a training set and a test (validation) set given a starting pool of compounds. In the training set-test set scenario, a model is developed using information contained only in the training set of compounds and this then validated by making predictions for the defined test set of compounds none of which were used to develop the model parameters. Activity classes are typically defined according to available binding strength ($K_i$, $IC_{50}$ etc) information. So, for example, active compounds may be defined to have $K_i \leq 10$ nM while inactives may be defined to have $K_i \geq 300$ nM. The precise thresholds used depend upon the data available and are defined by the user. However, it should be emphasized that the method is equally applicable to any situation where a binary classification of biological activity data can be defined. So, *in vivo* or *in vitro* measurements may be used which could be in the form of ADME (Absorption, Distribution, Metabolism, and Excretion), toxicity data and so on. For the sake of convenience, binding strength is used as an exemplifier of a biological end-point in order to illustrate the invention. The person of skill in the art will appreciate that other end points, including those mentioned above, are equally applicable and within the spirit and scope of the invention.

The DIV-DA discriminant method is inherently non-linear and, as explained below, unlike classical discriminant analysis (DA) [see, for example, Sharma, 1996] and methods such as recursive partitioning (RP) [Young and Hawkins, 1995; Chen *et al.,* 1998] does *not* rely only upon descriptors with large differences of means between classes. DIV-DA uses a novel descriptor selection method and a double filtering technique to select active compounds, again as described below. Furthermore, a means to generate a training and test set from a starting pool of available compounds can be built into the DIV-DA process.

In order to be efficient in the context of HTVS DIV-DA is typically based upon readily computed topological descriptors (*i.e.*, they are conformation-independent), but in principle this includes any computed or experimentally determined physical or chemical property of a molecule including 3D descriptors such as molecular fields [Cramer et al., 1988]. The only reason that 2D rather than 3D descriptors are currently preferred is the latter cannot currently be used for HTVS without exceptional computational resources.

A range of descriptors are defined in commercially available packages, such as TSAR™ software (Accelrys, US). Examples of descriptors are found in Grassy et al (1998) and in WO99/33598. Descriptors include 1D descriptors (e.g. molecular mass, counts of elements), 2D descriptors (e.g. counts of groups, topological, connectivity and shape indices) and 3D and 3D-sensitive descriptors (e.g. ellipsoidal volume, molar volume, lipophilicity, dipole moment, molecular fields) These and many other descriptors are defined in the "Handbook of Molecular Descriptors" (Todeschini & Consonni, 2000).

The number of descriptors in the ADS and IDS descriptor sets will be arrived at independently, and depend upon factors including the number of candidate descriptors evaluated, the number of active and inactive compounds used to evaluate the descriptors, and the particular nature of these compounds. Generally the number of candidate descriptors evaluated will be limited only by the number of descriptors readily available and which can be calculated in a convenient time frame and within the processing ability of the software and hardware available.

A small subset of descriptors will be selected by the method as sufficient to discriminate active from inactive compounds. A statistical rule-of-thumb is that the number of descriptors should be no more than approximately a quarter of the number of molecules in the training set. [Topliss and Edwards, 1979; Wold et al., 1983]. With additional descriptors the probability is increased of overfit of the model to the training data with a consequent lack of generalisability to other compounds, such as the virtual screening database molecules. For large training sets with perhaps some redundancy in terms of structural differences of the compounds we would prefer to keep the compound-descriptor ratio as small as possible. Thus, in the method application example given below the training set consisted of 321 compounds and it was found sufficient to have an ADS with seven descriptors and an IDS with ten descriptors - these are considered to be small sets of highly informative descriptors. We consider such compound-descriptor ratios to be typical and desirable for generalisation purposes.

A small subset of descriptors typically selected by a DIV-DA model from a training set with a range of different molecular scaffolds makes the method ideally suited to the aforementioned scaffold-hopping, as will be demonstrated below. For virtual screening purposes a training set should ideally present a maximum possible range of different molecular scaffolds. A minimum number might be considered to be three although there are no hard and fast rules about this since a limited number of scaffolds may have a wide variety of substituents thus providing a sufficient diversity of chemical information to build an informative screening model. If these criteria are met the method is eminently suitable for the identification of novel, potential lead structures. The compounds selected by virtual screening may be tested using HTS techniques or may be sufficiently small in number to be tested using traditional manual methods. Alternatively, prior to assay, and depending upon the information available, the selected structures may be passed through further, more time-consuming *in silico* filters such as a pharmacophoric or docking screen with a view to further enriching the selection.

A DIV-DA virtual screen may be used to identify a sub-set of compounds enriched with features selected by the model discriminant functions to favour strong binding to the chosen receptor. Various factors affecting binding strength may be implicit in a DIV-DA model, including pharmacophoric feature information and factors related to entropic and solvation/desolvation properties; the extent to which these various factors maybe described will depend upon the composition of the training set used to select a discriminant descriptor set.

Compound libraries which may be screened according to the invention may be any *in silico* collection of molecular structures. A number of such libraries are commercially available. Molecular structures may also be generated by de *novo* virtual synthesis, for example where one or a limited number of molecular pharmacophores are of particular interest.

The compound libraries which may be screened will typically contain structures representing small organic molecules, for example in the size range of 100 to 2000 Da. The number of molecules which may be screened is potentially unlimited and is only restricted by available computational resources. Desirably, a library of at least 1,000, preferably at least 10,000 and more preferably at least 100,000 compound structures is screened.

Thus a DIV-DA-based HTVS provides, at the very least, enrichment of a compound library for strong candidates. It is not essential or expected that, say, nanomolar level compounds will be immediately identified. In any case such strongly active compounds must, of course, be present in the screening database in order for them to be selected by any virtual screening method. As a general rule strong inhibitors are only likely to be identified after several iterations of (focussed) library design, virtual screening and assay. The advantage provided by DIV-DA is that it is expected to substantially reduce the number of compounds synthesized and tested thus reducing both the costs involved and the time required for lead identification.

DIV-DA models can of course be built and applied in an iterative manner. For example, an initial screen with DIV-DA and subsequent biological activity testing may identify a single molecular scaffold as interesting in terms of a given biological activity. A virtual library may then be built around this sole scaffold containing molecules not present in the original screening library. The existing DIV-DA model can then be used to virtual screen this new library. Alternatively, the DIV-DA model may be refined using the new molecules identified as active and inactive by the first biological screen. This DIV-DA model may then be used to screen the aforementioned single-scaffold library. This process of biological screening, model refinement and repeat virtual screening may be iterated many times until the objective is reached or no further improvements to the DIV-DA model can be made. DIV-DA involves the building of dual discriminant functions for the descriptive and predictive classification of strong active/weak-active or active/inactive compounds based upon analysis of descriptor distributions. The DIV-DA method may be fully automated within appropriate software. The method requires a pool of compounds with measured biological activity for the chosen biological target.

As alluded to in the preceding paragraph, the method of the invention discussed above may conveniently be implemented in software, for execution on any appropriate digital computer including one or more memory devices for storing the various data and one or more processors for executing the method.

Thus further aspects of the invention respectively provide a system (such as a computer or linked computers) operatively configured to implement a screening method of the invention in order to determine ADS and IDS sets for screening candidate compounds, or operatively configured with the ADS and IDS sets so determined together with the means to compare a database of compounds against these sets, and to provide an output identifying compounds which are likely active candidates.

Also included within the present invention are computer programming product or products (such as ROM, RAM, floppy discs, hard drives, optical compact discs, magnetic tapes, and other computer-readable media) carrying computer code for implementing the methods of the of the invention; and a computer program per se for implementing the method of the previous aspect of the invention.

Having outlined the invention in general terms, the following describes in more detail the steps of the preferred embodiment.

1.    Initial Steps

An initial dataset consists of compounds all assayed/tested against the chosen target and the available data must enable the identification of a binary classification such as strong active / weak active, active / inactive, toxic / non-toxic *etc*. There must be a clear separation between the activity values for the two classes and a minimum requirement is that this separation should exceed the experimental error associated with the chosen biological activity data. Typically, the separation will be much larger than this to ensure a clear biological activity "distance" between the two classes. In some cases it may be possible to optimise the thresholds such that a maximally predictive model is obtained (see validation below). In practise this has been found to be difficult to realise because of the lack of sufficient compounds with the requisite activity data, particularly for those with (very) weak binding strength which do not tend to be published. Compounds with activity values intermediate to the active-inactive cut-off thresholds are discarded at this stage.

The DIV-DA approach not only includes the selection of appropriate descriptors for classification but also provides a means to divide the initial pool of structures into a training and a test (validation) sets. Descriptors are calculated for all structures in the initial pool; this typically includes molecular attributes, molecular indices, atom, ring and group counts, 2D autocorrelograms and so on. In principle, any descriptors may be used, but 2D topological descriptors are preferred because, as noted above, the time required to process other types of descriptions (e.g. 3D conformational analysis and treatment of chiral compounds etc.) is prohibitive where many millions of structures are to be screened. This initial set of descriptors is usually subsequently statistically cleaned; this includes removal of those descriptors with zero or near zero variance, those with poor distribution, those highly intercorrelated and so on. If at all possible, a large, completely independent *screening* validation set should be identified so as to enable a realistic assessment to be made of the scaffold-hopping capability of the model; examples of such validation are given in the accompanying example.

2.    Descriptor Distribution and Discriminant Profile.

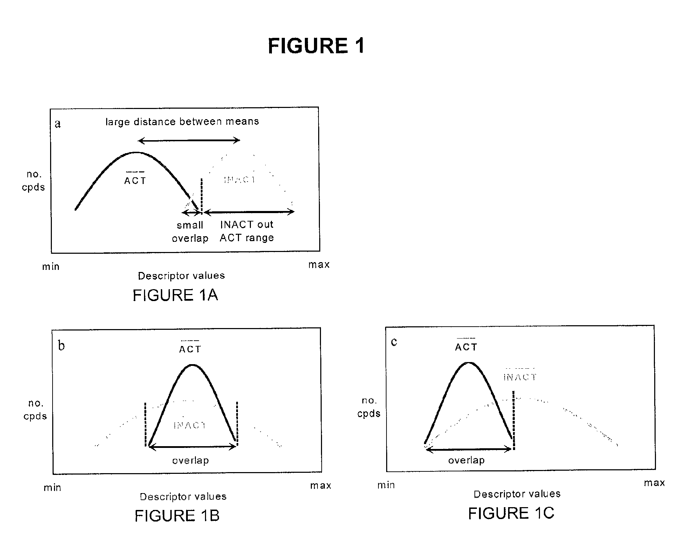

In a classical linear discriminant analysis (LDA) the preferred descriptors are those for which there is a large difference between means for the active and inactive compound distributions (Figure 1a). DIV-DA will also select descriptors with such properties (actives and inactives are clearly well separated) but in addition descriptors can also be selected which have much more overlapped or embedded distributions; some examples of such descriptor profiles are given in Figures 1b and 1c.

3.    Selection of Suitable Descriptors.

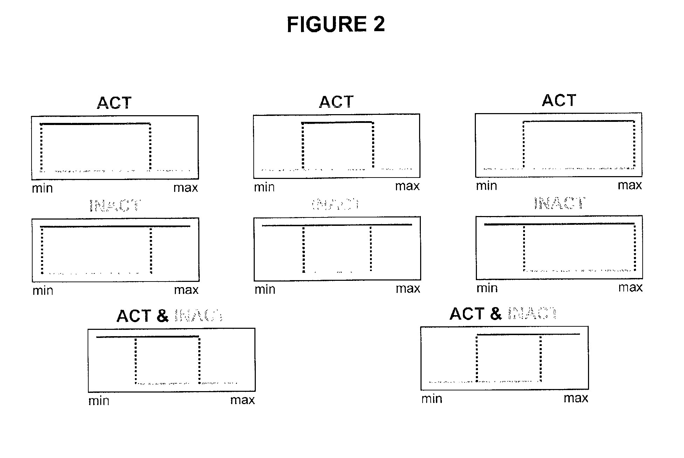

Once all available descriptors have been examined those descriptors suitable for inclusion in either the ADS or the IDS are selected using the following straightforward procedure. First a univariate analysis of descriptors is made determining maximum and minimum values for each descriptor for the active and inactive molecules separately and descriptor range overlaps identified. Descriptors suitable for inclusion in the ADS and/or IDS are then identified and these must conform to one of the seven active-inactive overlap profiles indicated in Figure 2.

Descriptors conforming to one of these profiles are included in the *candidate descriptor pool.* Profiles for descriptors without overlap are also considered even if this situation rarely occurs with large sets of compounds.

4.    Training and Test Set Specification.

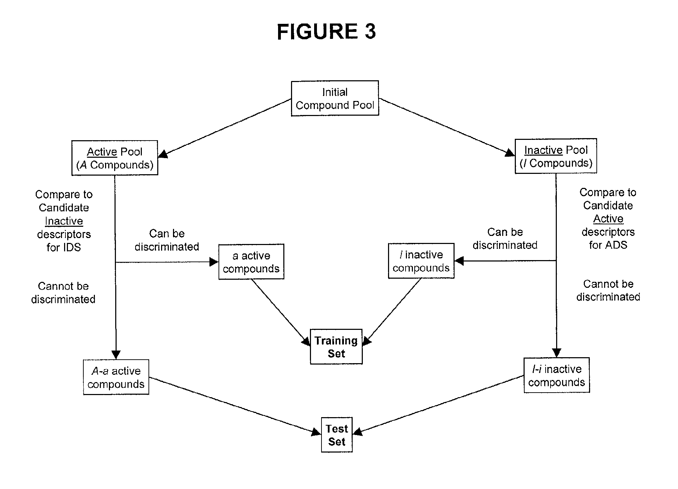

Prior to selecting which subset of the candidate descriptors is suitable for inclusion in a discriminant model the composition of the training and test sets is determined with reference to these descriptors; the process is outlined in Figure 3. At the outset the training set consists of *all* candidate molecules. Molecules are examined one by one and, on the basis of the following analysis method, are either retained in the training set or removed and placed in the test set. The method for doing this is as follows. Taking each class, active and inactive, separately molecules in one class are examined and scored on an individual basis according to the potential descriptors for the *other* class. So, for example, an active compound is examined with respect to each potential IDS descriptor (inactive descriptor) for the initial pool of compounds. If, for every one of these descriptors, this compound falls within the overlap region for active and inactive molecules then this compound can be also predicted inactive then it is not accepted as an active in the final analysis. This molecule is thus removed from the initial compound pool and placed in the test set. After examination of each molecule, a training and a test sets are obtained finally consisting of both active and inactive molecules. At the end of this process descriptor ranges and overlaps are recalculated. The next step is to select those descriptors to be included in the final ADS and IDS.

5.    Stepwise Procedure for Descriptor Selection.

Once the training and test sets have been defined descriptor selection can be made. From the candidate descriptor pool defined above, but subsequently modified as a result of the removal of test set compounds, the list of ADS and IDS candidate descriptors are updated and an overlap score is then determined; for an ADS candidate descriptor, is calculated as follows:

$$Overlap\_INACT = 100 \times \frac{\max(overlap) - \min(overlap)}{\max(INACT) - \min(INACT)}$$

whilst for an IDS candidate descriptor the overlap is calculated thus:

$$Overlap\_ACT = 100 \times \frac{\max(overlap) - \min(overlap)}{\max(ACT) - \min(ACT)}$$

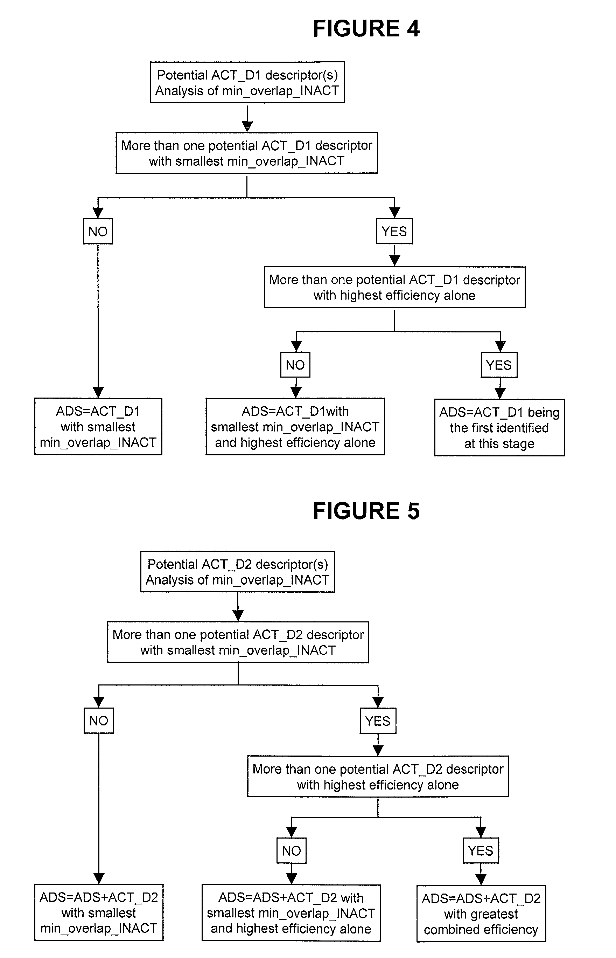

In this way descriptors for the ADS and IDS can be scored and then ranked on the basis that for a given descriptor the smaller the overlap score the more potentially interesting the descriptor. It is clear that in certain cases, such as where a classical DA distribution is present, a descriptor may be appropriate for inclusion in both the ADS and IDS. Where equal overlap scores arise for two or more descriptors then classification efficiency of the descriptors is considered; *i.e.*, the descriptor which allows to remove the maximal number of compounds of the opposite class is chosen. Figure 4 summarises the procedure used to select the first descriptor for inclusion in the ADS.

The process then proceeds in a stepwise manner adding descriptors in the sequence determined by the overlap score and separating those with equal overlap scores on the basis of classification efficiency (Figure 5). Each step begins by searching the smallest available min_overlap value and ends by discarding descriptors without efficiency to remove additional compounds of the opposite class for next step.

An analogous procedure is undertaken for selecting descriptors to be included in the IDS. For both the IDS and ADS independently the process is repeated until their is no further gain in the discriminatory effectiveness.

Thus, a final DIV-DA model consists of two sets of descriptors: the ADS and the IDS (the active and inactive descriptors sets, respectively). The quality of the model is judged both by training and test set classification performance.

Prediction can be made most efficiently for molecules belonging to the application domain. A molecule is then accepted as predicted active (some but preferably all the ADS descriptors are satisfied) only if it is not predicted to be inactive (at least, one IDS descriptor is not satisfied); in other words, DIV-DA is a double filtering technique.

One preferred method of classifying compounds from a set of compounds being screened is to select those members of the set which are within the application domain and then to rank these members using a score function, calculated by reference to both the number of ADS and IDS descriptors satisfied by each compound. A score function will allow the compounds of the set of compounds being screened to be ranked so that the members of the set most likely to be active can be selected for further analysis, such as testing. Various mathematical models can be used; for example one could calculate the ratio of percentage ADS descriptors satisfied to percentage IDS descriptors satisfied.

However, the most useful score function is one which provides a fixed range of from 0 to 1 (or 0% to 100%), independent of the number of ADS and IDS descriptors in a particular model (which would not be the case for the calculation of the preceding paragraph). For the compounds satisfying all the ADS descriptors a score function enables such molecules to be ranked according to how many of the IDS descriptors they satisfy, the fewer the better. For the molecules satisfying all the IDS descriptors the score function enables such compounds to be ranked according to how many of the ADS descriptors they satisfy. Thus a preferred score function may be calculated as:

$$score[cpd] = 0.5 \times \left( 1 - \left( \frac{nb\_ADS\_filters\_satisfied[cpd] - (2 \times \min A) + nb\_ADS\_filters\_model}{(\min A - nb\_ADS\_filters\_model)} \right) + \left( \frac{nb\_IDS\_filters\_satisfied[cpd] - (2 \times \min I) + nb\_IDS\_filters\_model}{(\min I - nb\_IDS\_filters\_model)} \right) \right)$$

where :

- nb_ADS_filters_satisfied[cpd] is the current number of ADS descriptors satisfied by the compound being ranked;
- nb_ADS_filters_model is the total number of ADS descriptors of the application domain;
- minA is the lowest number of ADS descriptors satisfied by any compound in the set being ranked which satisfies all the IDS descriptors;
- nb_IDS_filters_satisfied[cpd] is the current number of IDS descriptors satisfied by the compound being ranked;
- nb_IDS_filters_model is the total number of IDS descriptors of the application domain;
- minI is the lowest number of IDS descriptors satisfied by any compound in the set being ranked which satisfies all the ADS descriptors.

This formula provides a score function which will be from 0 to less than 0.5 for compounds predicted to be inactive, and from more than 0.5 to 1 for compounds predicted to be active.

The invention is illustrated by the following example, showing the effectiveness of the DIV-DA Approach for Identification of Novel HIV-1 Protease Inhibitors

Example.

1.      Initial database.

An initial database of molecular structures of HIV-1 PIs (HIV-1 protease inhibitors) was built using data from the abundant literature in this field. Active compounds were defined to have $K_I \leq 10$ nM (465 compounds) while inactive compounds were defined to have $K_I \geq 300$ nM (89 compounds). This separation is sufficiently large such that, even in the absence of the relevant data, it is expected to be significantly greater than experimental error for the $K_I$ values. Among the active compounds, there were the six marketed HIV PIs (amprenavir, indinavir, lopinavir, nelfinavir, ritonavir, saquinavir). The initial database contained more than 10 well known scaffolds of HIV PIs.

An initial set of 2D descriptors was calculated : classical topological indices [Molconn-Z], BCUT metrics [DiverseSolutions], counts of atoms pairs, and autocorrelograms ; the latter two descriptors are derived from the intramolecular distance matrix [proprietary tool].

After statistical cleaning of the first set of descriptors, the training set was created using DIV-DA: it contained 282 active and 39 inactive structures, with representatives of all the different scaffolds selected. Only one of the six marketed HIV PIs was in the training set.

DIV-DA analysis of the descriptors for the training set led to a model consisting of seven descriptors for ADS and ten descriptors for IDS. The training and test sets classification performance is given in table 1.

Table 1:

| Classification results of the training and test sets by DIV-DA model | | | | | | |
|---|---|---|---|---|---|---|
| Number of structures | Training Set | | Test Set | | Training and Test Sets | |
| | **true active** | true inactive | **true active** | true inactive | **true active** | true inactive |
| Starting point | **282** | 39 | **183** | 50 | **465** | 89 |
| Application domain | **282** | 39 | **183** | 50 | **465** | 89 |
| Predicted active | **282** | 0 | **152** | 24 | **434** | 24 |
| Predicted inactive | **0** | 39 | **15** | 13 | **15** | 52 |

The classification results for the training set were optimal : 100 % of correct classification for each class.

All the structures of the test set were within the application domain of the model.

The prediction of active structures for the test set led to the identification of 83 % of the true active compounds ; 13 % of predicted actives are false positives (24 inactive compounds selected with the 152 actives). This percentage of false positives is consistent with the lower level of information for inactive compounds (fewer structures) in the initial database ; such compounds are rarely described in the literature. Among the 152 active compounds identified by prediction of active structures, there were all the marketed ones (five compounds whereas the sixth was in the training set).

The prediction of inactive structures was not as efficient as the prediction of actives for the reasons explained above.

For the compounds of the active subset of the test set (prediction of active compounds is the objective in the present study), we observed that 83% were well classified, 8% were predicted inactive, only 2% were discarded as they were predicted simultaneously to be active and inactive, the remaining 7 % corresponded to compounds not satisfying all the ADS descriptors or not satisfying all the IDS descriptors.

Merging the training and the test sets, the percentage of compounds well classified by the model predictions is excellent for active compounds (93 %) and is reasonable for inactive compounds (58 %).

## 2. Screening of Public Compound Supplier Databases.

During the development of the DIV-DA method, a first training set was designed with a reduced number of three scaffolds in the initial database as other data were not yet available in the literature: pseudopeptides, cyclic ureas (1,3-diazepin-2-one derivatives) and monocyclic pyrones. This training set contained 22 active compounds and 25 inactive compounds. In the active subset, there were 5 marketed HIV PIs.

After DIV-DA analysis for model building, seven descriptors were selected for the ADS and nine descriptors for the IDS. In order to rapidly test the effectiveness of the method, this first model was used to screen a series of commercial supplier databases largely consisting of structures generated combinatorially (about 800,000 structures).

Due to biological assay facility limits (*in vitro* inhibition of HIV-1 protease), a selection of 60 compounds was made among the 1,667 structures predicted active by the DIV-DA model, and the measured kinetic parameter was $IC_{50}$ instead of $K_I$. Four of these compounds were found to be micromolar range inhibitors with $IC_{50}$ values of 2, 47, 52 and 59 μM (a fifth very weak inhibitor with an $IC_{50}$ of 363 μM was also detected - the 47 and 52 μM compounds are close analogues). The corresponding $K_I$ values were not determined but the equation of Cheng and Prusoff indicates that the $K_I$ values should be lower than $IC_{50}$ values [Cheng and Prusoff]. The structural features of these hits were very different from the scaffolds of the training set. Taking into account the results for the four micromolar inhibitors belonging to new scaffolds, the hit rate for the assayed compounds is 7 %.

In order to follow-up on these four compounds some further simple analysis was undertaken. Near neighbours (close analogues and further structures selected by similarity searching) were identified in the relevant original supplier databases and assays undertaken. The 2 μM compound appeared to be a false positive (giving a true hit rate of 5 %) while all analogues of the 363 μM compound exhibited no or equally weak activity. In contrast, activity identified for the two other new scaffolds was confirmed : a further twenty or so compounds were identified with activity in the range 30 to 100 μM ($IC_{50}$). These compounds provide a sound starting point for further SAR studies on original scaffolds and focussed library development.

## 3. Summary of screening results.

These results from screening public compound supplier databases demonstrate the successful validation of DIV-DA. There is a low expectation of finding a strongly active (say, nM level) HIV-1 PI in such databases; it is likely that these compounds will have been previously screened against HIV-1 protease and the best candidates removed from the databases. Furthermore, these databases are general purpose and have not (to our knowledge) been designed to be biased toward any particular target.

In addition, it should be noticed that it was not possible to assay all the predicted active compounds (only 60 out of 1,667 were selected for evaluation as HIV PIs). Furthermore, activity was reported as $IC_{50}$ rather than $K_I$ values. It is possible that $K_I$'s for the tested molecules is significantly lower than that suggested by the $IC_{50}$ values.

Nevertheless, the 5 % enrichment ratio observed after the first round of biological assays is greater than typical hit rates (0.1 to 1 %) for historical screening campaigns [Spencer, 1997]. Using the hit compounds and building focussed libraries (with or without the assistance of structure-based design), one might expect to find nanomolar level compounds.

## References.

Briem, H. and Lessel, U.T. *In Vitro* and *In Silico* Affinity Fingerprints: Finding Similarities Beyond Structural Classes. *Perspect. Drug Discov. Design,* 2000, 20, 231-244.

Brown, R.D. and Martin, Y.C. Use of Structure-Activity Data to Compare Structure-Based Clustering Methods and Descriptors for Use in Compound Selection. *J. Chem. Inf. Comput. Sci.,* 1996, 36, 572-584.

Brown, R.D. and Martin, Y.C. An Evaluation of Structural Descriptors and Clustering Methods for use in Diversity Selection. *SAR QSAR Environ. Res.*, 1998, 8, 23-39.

Chen, X., Rusinko III, A. and Young S. Recursive Partitioning of a Large Structure-Activity Data Set Using Three-Dimensional Decriptors. *J. Chem. Inf. Comput. Sci.,* 1998, 38, 1054-1062.

Cheng, Y and Prusoff, WH. Relationship between the inhibition constant ($K_I$) and the concentration of inhibitor which causes 50 per cent inhibition ($I_{50}$) of an enzymatic reaction. *Biochem. Pharmacol.,* 1973, 22, 3099-3108.

Cramer, R.D., Patterson, D.E. and Bunce, J.D. Comparative Molecular Field Analysis (CoMFA): 1. Effect of Shape on Binding of Steroids to Carrier Proteins. *J. Am. Chem. Soc.,* 1988, 110, 5959.

DiverseSolutions version 4.0, Tripos, St Louis, MO (information on DiverseSolutions is available at http://www.tripos.com/software/dvs.html - link last visited 09/Nov/01)

Dömling, A. and Ugi, I. Multicomponent Reactions with Isocyanides. *Angew. Chem. Int. Ed.,* 2000, 39, 3168-3210.

Dove, A. Drug Screening - Beyond the Bottleneck. *Nat. Biotechnol.*, 1999, 17, 859-864.

Estrada, E., Uriarte, E., Montero, A., Teijeira, M., Santana, L., De Clercq, E. A Novel Approach for the Virtual Screening and Rational Design of Anticancer Compounds. *J. Med. Chem.,* 2000, 43, 1975-1985.

Frimurer, T.M., Bywater, R., Nærum, L., Lauritsen, L.N. and Brunak, S. Improving the Odds in Discriminating "Drug-like" from "Non Drug-like" Compounds. *J. Chem. Inf. Comput. Sci.*, 2000; 40, 1315-1324.

Gálvez, J., García-Domenech, R., de Julián-Ortiz, J. V. and Soler, R. Topological Approach to Analgesia. *J. Chem. Inf. Comput. Sci.,* 1994, 34, 1198-1203.

Gálvez, J., García-Domenech, R., de Gregorio Alapont, C., de Julián-Ortiz, J. V. and Popa, L. Pharmacological Distribution Diagrams: A Tool for de Novo Drug Design. *J. Mol. Graph.,* 1996, 14, 272-276.

Gálvez, J.; García-Domenech, R.; Julián-Ortiz, J. V. de; Soler, R. Topological Approach to Drug Design. J. *Chem. Inf. Comput. Sci.,* 1995, 35, 272-284.

García-Domenech, R. and de Julián-Ortiz, J.V. Antimicrobial Activity Characterization in a Heterogeneous Group of Compounds. *J. Chem. Inf. Comput. Sci.,* 1998, 38, 445-449.

Grassy et al Nature Biotechnology 1998 Vol. 16 p748.

Grassy, G., Kaczorek, M., Lahana, R. and Yasri, A. Method for Providing, Identifying and Describing Molecules Capable of Having a Required Activity, in Particular in Pharmacology and Molecules Resulting from Said Method. International Patent WO 99/35598. July 15 1999.

Guner, O. (Ed.) Pharmacophore Perception, Development and Use in Drug Design. International University Line, 2000 Biotechnology Series. ISBN 0-9636817-6-1.

Hoffman B.T., Kopajtic, T., Katz, J.L. and Newman, A.H. 2D QSAR Modeling and Preliminary Database Searching for Dopamine Transporter Inhibitors Using Genetic Algorithm Variable Selection of MolConn Z Descriptors. *J. Med. Chem.,* 2000, 43, 4151-4159.

Hua Gao, Williams, S., Labute, P. and Bajorath, J. Binary Quantitative Structure-Activity Relationship (QSAR) Analysis of Estrogen Receptor Ligands. *J. Chem. Inf. Comput. Sci.*, 1999, 39, 164-168.

de Julián-Ortiz, J.V., Gálvez, J., Muoz-Collado, C., García-Domenech, R. and Gimeno-Cardona, C. Virtual Combinatorial Syntheses and Computational Screening of New Potential Anti-Herpes Compounds. *J. Med. Chem.,* 1999; 42, 3308-3314.

Labute, P. Binary QSAR: A New Method for the Determination of Quantitative Structure-Activity Relationships. *Biocomputing '99: Proceedings of the Pacific Symposium,* Altman, R.B., Lauderdale, K., Dunker, A.K., Hunter, L. and Klein, T.E. (Eds.) 1999, 4, pp 444-455, ISBN 981-02-3624-7.

Lipinski, C.A., Lombardo, L., Dominy, B.W. and Feeney P. J. Experimental and Computational Approaches to Estimate Solubility and Permeability in Drug Discovery and Development Settings. *Adv. Drug Deliv. Rev.,* 1997, 23: 3-25.

Matter H: Selecting Optimally Diverse Compounds from Structure Databases: a Validation Study of Two-Dimensional and Three-Dimensional Molecular Descriptors. *J. Med. Chem.,* 1997, 40, 1219-1229.

MOLCONN-Z version 3.5; Hall Associates Consulting, Quincy, MA (information on Molconn Z is available at http://www.eslc.vabiotech.com/molconn/ - link last visited 20/Dec/01).

NCI (National Cancer Institute) Database: available at http://www.dtp.nci.nih.gov/- link last visited 23/Jan/01.

Oprea, T. Property Distribution of Drug-Related Databases. *J. Comput.-Aid Mol. Design,* 2000, 14, 251-264.

Protherics Molecular Design Ltd.: http://www.protherics.com/ - link last visited 21/Mar/01.

Sharma, S. Applied Multivariate Techniques. John Wiley and Sons, Inc., 1996. ISBN 0-471-31064-6.

Spencer, R.W. Diversity Analysis in High-Throughput Screening. *J. Biomol. Screen.,* 1997, 2, 69-70.

Todeschini,R. and Consonni,V. (2000) Handbook of Molecular Descriptors. Mannhold, R.; Kubinyi, H. and Timmerman, H. (Eds) Vol 11 of Methods and Principles in Medicinal Chemistry, Wiley-VCH.

Topliss, J.G. and Edwards, R.P. (1979) Chance factors in studies of QSAR. *J. Med. Chem.,* 22, 1238-1244.

Walters, W.P., Ajay and Murcko, M.A. Recognizing Molecules with Drug-Like Properties. *Curr. Opin. Chem. Biol.,*

1999, 3, 384-387.

Walters, W.P., Stahl, M.T., Murcko, M.A. Virtual Screening - An Overview. *Drug Discov. Today*, 1998, 3, 160-178.

Wold, S., Johansson, E. and Cocchi, M., In: Kubinyi, H., (Ed.) 3D QSAR in Drug Design: Theory Methods and Applications, ESCOM, Leiden, The Netherlands, 1993, pp. 523-550.

Young, S.S. and Hawkins, D.M. Analysis of a Full $2^9$ Full Factorial Chemical Library. *J. Med. Chem.,* 1995, 38, 2784-2788.

**Claims**

1. A method for establishing a high-throughput virtual screening system, which method comprises:

   providing an initial database of molecular structures which comprise active compounds which exhibit a desired activity and inactive compounds which do not exhibit a desired activity;
   calculating descriptor values for a plurality of descriptors for said compounds; and
   selecting a set of descriptors, ADS, associated with compounds exhibiting said activity and a second set of descriptors, IDS, associated with compounds not exhibiting said activity, said second set of descriptors being different from said first set.

2. The method of claim 1 wherein said selecting is performed by:

   (a) providing a plurality of candidate active and inactive compound descriptors and defining a range of values of each descriptor for both active and inactive subsets of compounds;
   (b) dividing the initial database of compounds into a test set and a training set by a process which comprises;

      (1.i) evaluating each active compound by determining its descriptor value for each candidate inactive descriptor;
      (1.ii) selecting as a test set member each active compound whose descriptor value for each candidate inactive descriptor has a value which is in the range spanned by inactive compounds and selecting as a training set member the remaining active compounds; and
      (2.i) evaluating each inactive compound by determining its descriptor value for each candidate active descriptor;
      (2.ii) selecting as a test set member each inactive compound whose descriptor value for each candidate active descriptor has a value which is in the range spanned by active compounds and selecting as a training set member the remaining inactive compounds;

   (c) calculating the range of values for each descriptor of active and inactive compounds in the training set; and
   (d) selecting a plurality of descriptors and values thereof for the ADS and IDS based on the ability of the descriptors to discriminate active and inactive members of the training set.

3. The method of claim 2 wherein the descriptors to comprise the ADS descriptor set are selected on a stepwise basis in which each potential ADS descriptor is ranked for its degree of overlap with inactive compounds, a first descriptor with the least degree of overlap is selected, and the process is repeated to determine the next and subsequent descriptors until there is no further gain in their discriminatory effectiveness which allows removal of additional inactive compounds.

4. The method of claim 2 or 3 wherein the IDS descriptor set is selected on a stepwise basis in which each potential IDS descriptor is ranked for its degree of overlap with active compounds, a first descriptor with the least degree of overlap is selected, and the process is repeated to determine the next and subsequent descriptors until there is no further gain in their discriminatory effectiveness which allows removal of additional active compounds.

5. The method of claim 2, 3 or 4 which comprises validating the ADS and IDS descriptor sets by making active-inactive predictions for a plurality of compounds of the test set.

6. A method according to any one of the preceding claims wherein the initial compounds comprise a diversity of scaffolds.

7. A method according to any one of the preceding claims wherein the descriptors are 1D or 2D descriptors.

8. A method of screening a compound for a putative desired activity, which method comprises providing a screening system established according to the method of any one of claims 1 to 7, calculating for the compound its descriptors for the ADS and IDS sets, and selecting a compound as putatively having said desired activity if all the ADS descriptors are satisfied and, at least, one IDS descriptor is not satisfied.

9. A method according to claim 8 wherein the desired activity is selected from anti-viral activity, anti-bacterial activity, enzyme inhibitory activity, receptor-binding activity, biological macromolecule affinity where not covered by the previous two points, non-toxicity to a human, anticancer activity, and anti-pain activity.

10. A method according to claim 8, 9 or 10 wherein a library of at least 1000 compounds is screened.

11. A computer programming product carrying computer code for implementing the method of any one of claims 1 to 7.

12. A computer programming product carrying computer code representing selected ADS and IDS descriptor sets suitable for performing the method of any one of claims 8 to 10 for screening compounds for a desired activity.

13. A method according to any one of claims 8 to 10 which further comprises synthesising a compound predicted to be active for a desired activity.

14. A method according to claim 13 which further comprises testing the compound in a biological assay for its desired activity.

15. A method according to claim 13 or 14 which further comprises formulating the compound in the form of a pharmaceutical composition.

# FIGURE 1

**a**

large distance between means

$\overline{ACT}$

INACT

no. cpds

small overlap

INACT out ACT range

min                                          max

Descriptor values

FIGURE 1A

**b**

$\overline{ACT}$

INACT

no. cpds

overlap

min                                          max

Descriptor values

FIGURE 1B

**c**

$\overline{ACT}$

INACT

no. cpds

overlap

min                                          max

Descriptor values

FIGURE 1C

# FIGURE 2

# FIGURE 3

EP 1 326 183 A1

# FIGURE 4

Potential ACT_D1 descriptor(s)
Analysis of min_overlap_INACT

→

More than one potential ACT_D1 descriptor
with smallest min_overlap_INACT

NO

YES

More than one potential ACT_D1 descriptor
with highest efficiency alone

NO

YES

ADS=ACT_D1
with smallest
min_overlap_INACT

ADS=ACT_D1with
smallest min_overlap_INACT
and highest efficiency alone

ADS=ACT_D1 being
the first identified
at this stage

# FIGURE 5

Potential ACT_D2 descriptor(s)
Analysis of min_overlap_INACT

→

More than one potential ACT_D2 descriptor
with smallest min_overlap_INACT

NO

YES

More than one potential ACT_D2 descriptor
with highest efficiency alone

NO

YES

ADS=ADS+ACT_D2
with smallest
min_overlap_INACT

ADS=ADS+ACT_D2 with
smallest min_overlap_INACT
and highest efficiency alone

ADS=ADS+ACT_D2
with greatest
combined efficiency

**EP 1 326 183 A1**

## EUROPEAN SEARCH REPORT

**European Patent Office**

**Application Number**

EP 02 29 0037

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A,D | WO 00 79263 A (SYNT EM S A) 28 December 2000 (2000-12-28) * the whole document * | 1 | G06F17/30 G06F19/00 |
| A,D | FR 2 773 240 A (SYNT EM) 2 July 1999 (1999-07-02) * the whole document * | 1 | |
| A | BAUMANN K: "Uniform-length molecular descriptors for quantitative structure-property relationships (QSPR) and quantitative structure-activity relationships (QSAR): classification studies and similarity searching" TRAC, TRENDS IN ANALYTICAL CHEMISTRY, ANALYTICAL CHEMISTRY. CAMBRIDGE, GB, vol. 18, no. 1, January 1999 (1999-01), pages 36-46, XP004159338 ISSN: 0165-9936 * the whole document * | 1 | |
| A | TOLEDO-SHERMA L M ET AL: "HIGH-THROUGHPUT VIRTUAL SCREENING FOR DRUG DISCOVERY IN PARALLEL" CURRENT OPINION IN DRUG DISCOVERY AND DEVELOPMENT, CURRENT DRUGS, LONDON, GB, vol. 5, no. 3, May 2000 (2000-05), pages 414-421, XP001088058 ISSN: 1367-6733 * the whole document * | 1 | **TECHNICAL FIELDS SEARCHED (Int.Cl.7)** G06F |

The present search report has been drawn up for all claims

| Place of search | Date of completion of the search | Examiner |
|---|---|---|
| THE HAGUE | 7 November 2002 | DE CASTRO PALOM.., L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

20

**ANNEX TO THE EUROPEAN SEARCH REPORT
ON EUROPEAN PATENT APPLICATION NO.** EP 02 29 0037

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2002

| Patent document cited in search report | | Publication date | Patent family member(s) | | Publication date |
|---|---|---|---|---|---|
| WO 0079263 | A | 28-12-2000 | AU | 4565600 A | 09-01-2001 |
| | | | WO | 0079263 A2 | 28-12-2000 |
| FR 2773240 | A | 02-07-1999 | FR | 2773240 A1 | 02-07-1999 |
| | | | AU | 1972299 A | 26-07-1999 |
| | | | CA | 2315364 A1 | 15-07-1999 |
| | | | EP | 1044418 A1 | 18-10-2000 |
| | | | WO | 9935598 A1 | 15-07-1999 |
| | | | JP | 2002501233 T | 15-01-2002 |

EPO FORM P0459

For more details about this annex : see Official Journal of the European Patent Office, No. 12/82